# EUROPEAN PATENT SPECIFICATION

(11) **EP 3 982 891 B1**
(45) Date of publication and mention of the grant of the patent: **20.03.2024**
(21) Application number: 19932610.9
(22) Date of filing: 11.06.2019
(51) Int. Cl.: A61F 5/455

(54) **MENSTRUAL CUP FORMED OF RENEWABLE THERMOPLASTIC ELASTOMER AND A METHOD FOR MANUFACTURING SUCH A CUP**
AUS EINEM ERNEUERBAREN THERMOPLASTISCHEN ELASTOMER GEBILDETE MENSTRUATIONSTASSE UND VERFAHREN ZUR HERSTELLUNG EINER SOLCHEN TASSE
COUPE MENSTRUELLE FORMÉE D'ÉLASTOMÈRE THERMOPLASTIQUE RENOUVELABLE ET PROCÉDÉ DE FABRICATION D'UNE TELLE COUPE

(43) Date of publication of application: 20.04.2022
(73) Proprietor: Essity Hygiene and Health Aktiebolag, 405 03 Göteborg (SE)
(72) Inventor: ALENIUS, Karin, 405 03 Göteborg (SE)
(74) Representative: Nederlandsch Octrooibureau
(86) International application number: PCT/SE2019/050549
(87) International publication number: WO 2020/251433

(56) References cited:
- EP-A1- 0 045 790
- EP-A2- 2 424 937
- WO-A1-2016/160343
- WO-A1-2017/015767
- WO-A1-2019/020958
- WO-A1-2019/020958
- DE-U1-202009 008 893
- DE-U1-202009 008 893
- US-B2- 9 827 136
- "Pearlthane ECO D12T85", Technical Data Sheet, 2018, XP055769176, Retrieved from the Internet: URL:https://www.lubrizol.com/Engineered-Po lymers/Products/Pearlthane-ECO-TPU [retrieved on 2020-02-28]
- "Dryflex Green, Soft plastics from plants", Technical Data Sheet, 5 October 2015 (2015-10-05), XP055769179, Retrieved from the Internet: URL:https://www.hexpoltpe.com/en/dryflex-g reen.htm [retrieved on 2020-02-28]

## Description

### TECHNICAL FIELD

The present disclosure relates to a menstrual cup adapted for the collection of menstrual fluids and methods for the manufacturing and use thereof. WO 2019/020958 A1 discloses the features of the preamble of claims 1, 13 and 15.

### BACKGROUND

In the current society, consumers are increasingly aware of the environmental effects of their purchases. This often results in a feeling of guilt when purchasing products having a short usage time, such as a couple of hours, made from materials originating from finite sources such as disposable articles. Such feelings are often referred to as "consumer guilt".

Menstrual cups having a longer usage time can partly relief this consumer guilt. Such menstrual cups have previously been known in the art by, for example, international patent application WO2006058409 A1.

However, many menstrual cups produced today are manufactured from organosilicon oxide polymers wherein the hydrocarbons originate from non-renewable resources such as petroleum or natural gas. In addition to the limited supply of petroleum and natural gas, the methods for obtaining and processing these materials have a negative impact on the environment.

Therefore, there is an increased need for the development of menstrual cups manufactured from materials with an improved environmental impact.

### SUMMARY

The present disclosure is based on the need for menstrual cups manufactured from materials with an improved environmental impact. The present disclosure provides a menstrual cup comprising a renewable thermoplastic elastomer.

The disclosure concerns a non- absorbent article according to claims 1 to 9, an array according to claims 10-12 and a method according to claims 13 and 14, and a use according to claim 15. Further embodiments are set out in the dependent claims, in the following description and in the drawings.

At least one of the objectives is achieved by, or at least partly achieved by, a menstrual cup comprising a receptacle. The receptacle has an exterior surface and an interior surface, with the interior surface defining at least in part an interior cavity adapted for collecting fluid. The receptacle extends from an open top end to a closed bottom end, wherein the open top end has a predetermined diameter. The receptacle further comprises an upper rim portion at the open top end of the receptacle, and a main portion defining a decreasing diameter of the interior cavity while extending downwards from the open top end to the closed bottom end of the receptacle. Furthermore, the receptacle is at least partly formed of a renewable thermoplastic elastomer.

Thermoplastic elastomers have high elastomeric properties giving the menstrual cup the required flexibility to assume different configurations for easy inserting and removing of the menstrual cup, and to return to its original configuration during use. Furthermore, thermoplastic elastomers are easy to use in manufacturing, especially in injection molding processing. Due to their thermoplastic properties, thermoplastic elastomers can undergo several cycles of molding, extrusion and use and can thus be recycled. This is in contrast to rubbers which are in general not recyclable due to their thermosetting characteristics.

The use of a renewable thermoplastic elastomer in the manufacturing of the menstrual cup reduces the environmental impact of the menstrual cup manufacturing process. Further, the use of a renewable thermoplastic elastomer for the manufacturing of the menstrual cup may partly relief consumer guilt when discarding the menstrual cup.

The thermoplastic elastomeric material may be selected from the thermoplastic polyurethane elastomers and/or thermoplastic styrene elastomers. Thermoplastic polyurethane elastomers have a higher Shore A value than thermoplastic styrene elastomers. However, thermoplastic styrene elastomers have a lower tear strength than thermoplastic polyurethane elastomers.

The renewable thermoplastic elastomer may be present at about 20-100% by weight of the total weight of the thermoplastic elastomer.

The renewable thermoplastic elastomer may be present at about 30-90% by weight of the total weight of the thermoplastic elastomer.

The renewable thermoplastic elastomer may be present at about 40-80% by weight of the total weight of the thermoplastic elastomer.

The renewable thermoplastic elastomer may be present at about 50-70% by weight of the total weight of the thermoplastic elastomer.

The renewable polymer in the present disclosure may not be biodegradable as biodegradable polymers have hydrophilic properties which may hinder effective containment of the menstrual fluid in the receptacle.

The main portion of the menstrual cup may have a shore hardness between Shore A 20 and Shore A 70 to provide sufficient flexibility and resiliency for the menstrual cup to assume different configurations pre-use, during insertion/removal and during use.

The menstrual cup may be a one-period cup. By the term "one-period cup" as used herein, is meant a cup that can be discarded after one menstrual period while at the same time having a limited negative impact on the environment due to the presence of renewable polymers in the menstrual cup. This may also lead to a reduced feeling of "consumer guilt". By reducing the usage time of the menstrual cup to one menstrual period, there may be a reduced requirement for cleaning and sterilization.

An array of menstrual cups may be provided both in respect of receptacle dimension and Shore A hardness of the material. Thus, an array may be provided wherein the receptacle has at least two different dimensions such that the first dimension has the capacity to receive a larger amount of fluid than the second dimension. By providing menstrual cups with different dimensions, menstrual cups can be provided to users with different vaginal anatomies. For example, a menstrual cup of a smaller size might be a better fit for women having a low cervix and thus shorter vaginal canal.

Conversely, a menstrual cup having a bigger size might be a better fit for women having a high cervix and thus longer vaginal canal. Furthermore, an array of menstrual cups may be provided wherein the receptacle has at least two different dimensions such that the first dimension is adapted for uses that have not given birth vaginally and a second dimension adapted for users that have given birth vaginally. As childbirth is often accompanied by physical modification in the vaginal channel, it may be beneficial to adapt the menstrual cup to these physical modifications by e.g. changing the dimension of the receptacle. Further, an array of menstrual cups may be provided wherein the material constituting the receptacle has at least two Shore A values depending on the user preferences. In general, softer menstrual cups are more comfortable during use as they apply less outward pressure against the vaginal wall. On the other hand, softer menstrual cups require more manual manipulation to get them to open inside the vagina. Further, use of harder menstrual cups may be recommended during exercise as these cups will be less prone to being pushed out by the muscles in the vaginal canal.

By providing the receptacles of varying dimensions and/or material hardness as an array, it is clear to the user that the menstrual cups are produced by the same manufacturer, and therefore the same quality level is to be expected.

The present disclosure also pertains to a method for manufacturing a menstrual cup comprising a first step of designing a menstrual cup comprising a receptacle having an exterior surface and an interior surface, the interior surface defining at least in part an interior cavity adapted for collecting fluid, the receptacle further extending from an open top end to a closed bottom end, the open top end having a predetermined diameter, the receptacle further comprising an upper rim portion at the open top end of the receptacle, and a main portion defining a decreasing diameter of the interior cavity while extending downwards from the open top end to the closed bottom end of the receptacle; a second step of obtaining mold tooling configured for the menstrual cup design; a third step of molding the menstrual cup with the mold tooling; and a fourth step of extracting the menstrual cup from the molding. The manufacturing method further comprises the step of manufacturing the menstrual cup receptacle so that it comprises a renewable polymer.

According to the present disclosure, the method for manufacturing the menstrual cup may furthermore comprise the step of selecting a thermoplastic elastomeric material selected from thermoplastic polyurethane elastomers and/or thermoplastic styrene elastomers.

The disclosure also pertains to the use of thermoplastic elastomeric material selected from thermoplastic polyurethane elastomers and/or thermoplastic styrene elastomers for manufacturing a one-period cup.

### BRIEF DESCRIPTION OF THE FIGURES

The present disclosure will be further explained hereinafter by means of examples and with reference to the appended drawings wherein:
- Fig. 1: shows a side view of the menstrual cup;
- Fig. 2: shows a side view of a further example of the menstrual cup;
- Fig. 3: shows a top view of the menstrual cup;

### DETAILED DESCRIPTION

A "menstrual cup" is a cup-shaped device to be inserted into the vagina for the reception and containment of menstrual fluid.

The term "renewable material" refers to material composed of biomass from a living source and that can be continually replenished in a rate equal to or greater than the rate of depletion, for example wood, crops, marine products or organic waste.

"Biodegradable material" refers to material that rapidly decomposes by microorganisms and other biological processes. Biodegradation is a chemical process in which materials, with the help of microorganisms, are metabolized to water, carbon dioxide, and biomass.

"Finite sources" relates to resources that are non-renewable such as carbon-based fossil.

As used in this disclosure, "array" refers to a display of packages comprising menstrual cups of different receptacle dimension and/or material hardness but having a similar construction. The packages have the same brand and/or sub-brand and may also have the same trademarks. The packaging elements such as material type, dominant color and/or design) are similar to convey to the customer that the different individual packages are part of a larger line-up. The packages are placed in proximity to each other in a given area in a retail store.

In all figures in the following detailed description, the same reference numerals will be used to indicate the same elements.

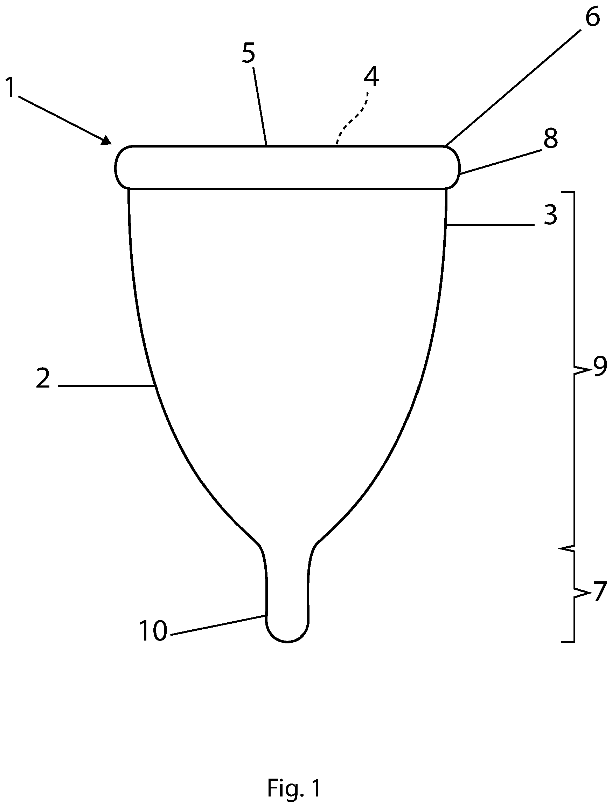

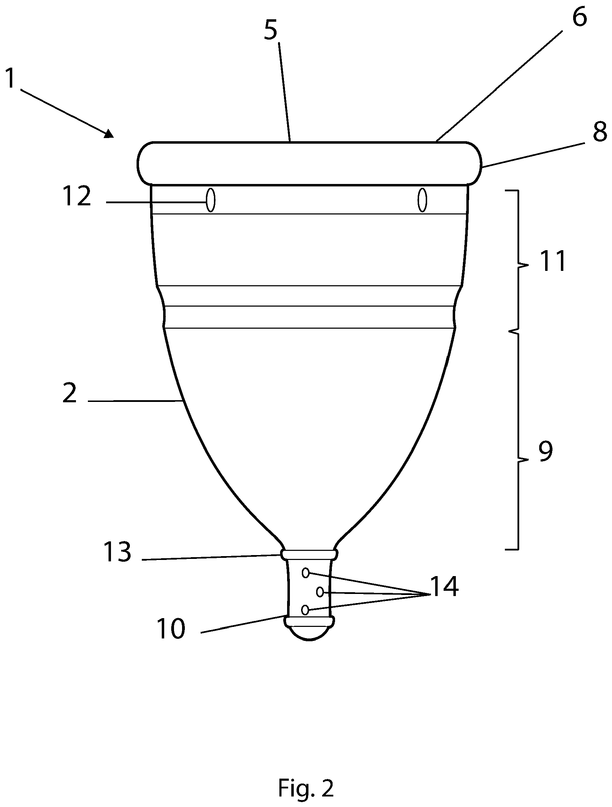

Figure 1 shows a side view of the menstrual cup according to the present disclosure. The cup 1 comprises a receptacle 2 having an exterior surface 3 and an interior surface 4, the interior surface 4 defining an interior cavity 5 for the collection of menstrual fluid. The receptacle 2 extends from an open top end 6 to a closed bottom end 7. The open top end 6 comprises an upper rim portion 8 with a predefined diameter. The receptacle 2 further comprises a grip 10 extending from the closed bottom end 7 for easy removal of the menstrual cup 1. Furthermore, the receptacle 2 has a main portion 9 defining a decreasing diameter of the interior cavity 5 while extending downwards from the open top end 6 to the closed bottom end 7. The main portion 9 may comprise one or more ridges. As shown in figure 2, the menstrual cup may additionally comprise a transition portion 11 located between the upper rim portion 8 and the main portion 9.

The menstrual cup according to the present disclosure may have at least two configurations; a folded configuration for inserting/removing the menstrual cup into the vagina and a deployed configuration during use. The folded configuration is achieved by folding the resilient material of the receptacle 2 along the longitudinal axis. The menstrual cup is inserted into the vagina in a folded configuration with the open top end facing the cervix thus exposing the interior cavity 5 to the cervix for the collection of fluids. Once inserted, the menstrual cup assumes its deployed configuration with the upper rim portion 8 assuming its original circular perimeter to form a seal between the menstrual cup 1 and the vaginal wall.

In figure 2, the transition portion 11 is provided with apertures 12 which may be located in the vicinity of the upper rim portion 8. Alternatively, the apertures may be present in the main portion 9. The apertures serve to equalize the air pressure inside and outside the receptacle 2 during insertion and removal of the menstrual cup. Failure to equalize air pressure could result in a suctional force directed towards the vagina during removal of the menstrual cup. In the deployed configuration, the apertures 12 are sealed off by the vaginal wall thus allowing the creation of a natural suction force between the menstrual cup and the vaginal wall for keeping the menstrual cup in place. As shown in figure 2, the transition zone may have one or more ridges 13. Alternatively, the transition portion 11 may be substantially smooth or have knob-like protrusions 14.

The menstrual cup can be removed from the vagina by pulling the grip 10 extending from the closed bottom end 7. As shown in figure 2, the grip 10 may be provided with extra ridges 13 and/or protrusions 14 to make it easier for the user to grasp the grip 10. The ridges 13 may be present in the form of a series of radial ridges. The grip 10 may be hollow inside to make the grip less sturdy thus increasing comfort during insertion and removal. The grip 10 forms an integral part of the receptacle 2 and is therefore not detachable. In figure 1 and figure 2, the grip 10 has a cylindrical shape. However, the grip may also be formed as a knob having a rounded shape, or as a rectangle, or any other suitable form for easy handling of the grip. Further, the grip may be formed as a long oblong shape divided into different sections, so that the grip may be trimmed to the desired length by the user.

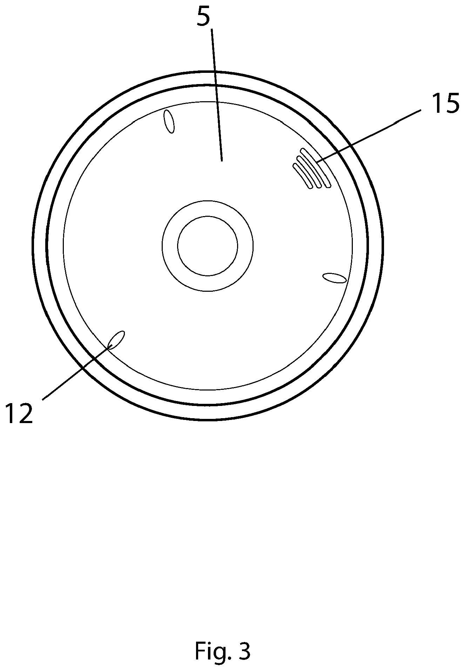

Figure 3 shows a top view of the menstrual cup further comprising a volume marking 15 positioned on the inner wall surface of the receptacle 2. The volume marking informs the user about the volume of fluid that has accumulated in the receptacle 2. This information may give the user an indication about the heaviness of the menstrual flow. Depending on the geographical location where the user is located, and the measurement units used in that specific area, the volume marking may be expressed in imperial units or in metric units. The volume markings may also be expressed in both imperial and metric units.

The material thickness in the receptacle 2 varies between the different defined portions within the menstrual cup 1. The material thickness in the upper rim portion 8 is greater than the material thickness in the main portion 9 and in the transition portion 11, thus providing the upper rim portion 8 with rigidity for maintaining the menstrual cup in a pre-determined position in the vagina. The material thickness in the transition portion 11 may be greater than in the main portion 9 resulting in an increased sturdiness of the transition portion to support the upper rim portion 8 in retaining the pre-determined shape of the menstrual cup. The material thickness in the receptacle 2 may decrease downwards from the upper rim portion 8 to the closed bottom end 7. The material thickness in the upper rim portion 8 may be about 5 mm. The material thickness in the transition portion may decrease from about 3 mm at the end of the transition portion adjacent to the upper rim portion 8, to about 2 mm at the end of the transition portion adjacent to the main portion. The material thickness in the main portion 9 may be about 2 mm.

The material thickness can be measured using the following method: thickness is measured on the main portion (reference numeral 9 in fig. 1 or fig 2), i.e. on a representative (large) area that is free from rims or other irregularities. The menstrual cup may have to be cut to lay bare this area. A digital caliper (available e.g. from Mitutoyo, Japan) is utilized. The reading is taken just when the jaws touch the cup wall (i.e. no significant pressure is applied). In case the main portion has a varying thickness, a number of readings are taken to obtain a representative mean.

According to the present disclosure, an array of menstrual cups may be constructed wherein the receptacle has different dimensions corresponding to varying capacities for receiving liquid. For example, a first article may have a smaller liquid receiving capacity, e.g. 20 ml, a second article may have a larger liquid receiving capacity, e.g. 25 ml. The array may also include a third article having a largest liquid receiving capacity of e.g. 30 ml. The array of menstrual cups may also be constructed such that a first dimension of the receptacle is adapted for users that have not given vaginal birth and a second dimension of the receptacle is adapted for users that have given vaginal birth. The different dimensions of the receptacle may enable the array of menstrual cups to accommodate the physical modifications that are associated with child birth. In general, the dimensions of the receptacle may cover a range of 15 to 50 ml.

Further, an array of menstrual cups may be provided wherein the material constituting the receptacle has at least two Shore A values depending on the user preferences. For example, a first menstrual cup may have a Shore A value within the range of 20 to 30, while a second menstrual cup has a Shore A value within the range of 40 to 50. The array may further comprise a third menstrual cup having a Shore A value between 50 and 70.

An important aspect of the current disclosure is that the production of the menstrual cup has a decreased environmental impact as compared to menstrual cups produced entirely from materials originating from finite sources. Figure 1 shows a side view of the menstrual cup according to the present disclosure. As the receptacle 2 comprises renewable material, the originating source is replenished in a rate equal to or greater than the rate of depletion. Standardized analytical methods are available for determining the quantity of renewable material in the menstrual cup. For example, ASTM D6866 which uses radiocarbon dating and is offered by specialized testing laboratories.

The material constituting the receptacle 2 may be a thermoplastic elastomer to provide the necessary flexibility for the menstrual cup to assume an optimal fit during use and to enable easy folding and deployment. The renewable material in the menstrual cup may be selected from the thermoplastic polyurethane elastomers and/or thermoplastic styrene elastomers. Examples of renewable elastomers are Dryflex^{®} Green by Hexpol TPE and bio TPU^{™} by Lubrizol. Because renewable thermoplastic elastomers are generally harder and less flexible, it may be advantageous to use a blend of renewable and non-renewable elastomers in the receptacle 2 of the menstrual cup. Thus, the amount of renewable polymer may be present at about 20-100% by weight of the total weight of the thermoplastic elastomer. The renewable thermoplastic elastomer may also be present at about 30-90% by weight of the total weight of the thermoplastic elastomer, 40-80% or 50 to 70% by weight of the total weight of the thermoplastic elastomer. The entire menstrual cup may be constructed from the same material. The thermoplastic elastomer is preferably a medical grade. Because of the health-related risks associated with latex allergies (WU et al. Current prevalence rate of latex allergy: Why it remains a problem? In: Journal of Occupational Health 2016, 58(2): 138-144), the use of latex as a renewable polymer is outside the scope of the current disclosure.

Furthermore, the renewable thermoplastic elastomer in the present disclosure may not be biodegradable as biodegradable polymers have hydrophilic properties which may hinder effective containment of the menstrual fluid in the receptacle.

As described earlier, the material in the menstrual cup wall may be flexible and can therefore easily adapt different configurations for optimal fit into the vagina and for easy insertion and removal. The material may be resilient, so that the menstrual cup restores to its original shape after being compressed. The material hardness in the main portion of the menstrual cup wall may be between a Shore A value of 20 and a shore A value of 70, to provide the required flexibility and resiliency.

The hardness of the material constituting the menstrual cup can be determined through the following method: hardness of the wall material can be quantified in Shore hardness units. The determination is done according to ISO 868 (2003 version), Plastics and ebonite - Determination of indentation hardness by means of a durometer (Shore hardness). The Shore A scale is utilized. A suitable tester is available from Kern, Germany, under the designation Sauter HDA 100-1 (Shore A). The instrument is held against the test object, and an indenter is forced into the object. The hardness is inversely related to the penetration depth of the indenter. Shore hardness is reported on scale ranging from 0 to 100, where higher figures represent harder materials. For the evaluation, the menstrual cup is removed from its package, and is then conditioned for 24 hours in a laboratory environment set to 23°C and 50% relative humidity. Hardness is measured on the main portion (reference number 9 in figure 1 or figure 2), i.e. on a representative (large) area that is free from rims or other irregularities. The material must be at least 4 mm thick for proper evaluation. If the cup wall is thinner, pieces are cut from the main portion, and the pieces are stacked to obtain a total thickness of at least 4 mm. The convex side of the stack should face the tabletop underneath, and when applying the instrument from above enough pressure should be applied to flatten out the concavity around the indenter. The hardness value is read after 3 seconds. Five measurements are made on three different menstrual cups, and the mean from the 15 individual tests is reported.

The menstrual cup may be manufactured by injection molding the wall material in the desired unfolded configuration. The mold tooling may consist of several parts that need to be disassembled after the molding step to reveal the menstrual cup end product. Trimming may be required to remove excess material not forming part of the menstrual cup. Alternatively, vacuum forming, die casting, blow molding or extrusion may be used for shaping the wall material in the desired configuration.

## Claims

1. A menstrual cup (1) comprising a receptacle (2) having an exterior surface (3) and an interior surface (4), the interior surface (4) defining at least in part an interior cavity (5) adapted for collecting fluid, the receptacle (2) further extending from an open top end (6) to a closed bottom end (7), the open top end (6) having a predetermined diameter, the receptacle (2) further comprising an upper rim portion (8) at the open top end (6) of the receptacle (2), and a main portion (9) defining a decreasing diameter of the interior cavity (5) while extending downwards from the open top end (6) to the closed bottom end (7) of the receptacle (2), said receptacle (2) being made from a thermoplastic elastomer **characterized in that**
said thermoplastic elastomer comprises an amount of renewable thermoplastic elastomer, defined as an elastomer that is originating from biomass from a living source.

2. Menstrual cup (1) according to any of the preceding claims, wherein said renewable thermoplastic elastomer is selected from polyurethane elastomers and/or styrene elastomers.

3. Menstrual cup according to claim 1 or 2, wherein said renewable thermoplastic elastomer is present at about 20-100% by weight of the total weight of the thermoplastic elastomer.

4. Menstrual cup (1) according to claim 3, wherein said renewable thermoplastic elastomer is present at about 30-90% by weight of the total weight of the thermoplastic elastomer.

5. Menstrual cup (1) according to claim 4, wherein said renewable thermoplastic elastomer is present at about 40-80% by weight of the total weight of the thermoplastic elastomer.

6. Menstrual cup (1) according to claim 5, wherein said renewable thermoplastic elastomer is present at about 50-70% by weight of the total weight of the thermoplastic elastomer.

7. Menstrual cup according to any of the preceding claims, wherein said renewable thermoplastic elastomer is not biodegradable.

8. Menstrual cup according to any of the preceding claims, wherein said main portion (9) of the receptacle (2) has a shore hardness between Shore A 20 and Shore A 70 determined by ISO 868 (2003 version).

9. Menstrual cup (1) according to any of the preceding claims, wherein the thermoplastic elastomer does not contain latex.

10. An array of menstrual cups according to any of the preceding claims, wherein said array comprises at least two receptacles (2) having different dimensions such that a first of the at least two receptacles (2) has the capacity to receive a larger amount of fluid than a second of the at least two receptacles (2).

11. An array of menstrual cups according to any of the preceding claims, wherein said array comprises at least two receptacles (2) having different dimensions such that a first of the at least two receptacles (2) has dimensions that are adapted for users that have a low cervix and shorter vaginal canal and a second of the at least two receptacles (2) has dimensions that are adapted for users that have a high cervix and a longer vaginal canal.

12. An array of menstrual cups according to any of the preceding claims, wherein said array comprises at least two receptacles (2) having different levels of material hardness such that a first of the at least two receptacles (2) has a material hardness level that is higher than a material hardness level of a second of the at least two receptacles (2).

13. A method for manufacturing a menstrual cup (1) having a design comprising a receptacle (2) having an exterior surface (3) and an interior surface (4), the interior surface (4) defining at least in part an interior cavity (5) adapted for collecting fluid, the receptacle (2) further extending from an open top end (6) to a closed bottom end (7), the open top end (6) having a predetermined diameter, the receptacle (2) further comprising an upper rim portion (8) at the open top end (6) of the receptacle (2), and a main portion (9) defining a decreasing diameter of the interior cavity (5) while extending downwards from the open top end (6) to the closed bottom end (7) of the receptacle (2), the method comprising:
molding the menstrual cup (1) with a mold tooling configured for the menstrual cup design, using a thermoplastic elastomer; and
extracting the menstrual cup (1) from the molding;
**characterized in that**
the thermoplastic elastomer used for molding the menstrual cup (1) comprises an amount of renewable thermoplastic elastomer, defined as a thermoplastic elastomer that is originating from biomass from a living source.

14. Method according to claim 13, wherein said renewable thermoplastic elastomer is selected from polyurethane elastomers and/or styrene elastomers.

15. Use of thermoplastic elastomeric material for manufacturing a menstrual cup, **characterized in that** said thermoplastic elastomeric material comprises an amount of renewable thermoplastic elastomer, defined as a thermoplastic elastomer that is originating from biomass from a living source, said renewable thermoplastic elastomer being selected from polyurethane elastomers and/or styrene elastomers.

## Patentansprüche

1. Menstruationstasse (1), umfassend einen Behälter (2) mit einer Außenfläche (3) und einer Innenfläche (4), wobei die Innenfläche (4) zumindest teilweise einen inneren Hohlraum (5) definiert, der zum Sammeln von Flüssigkeit geeignet ist, wobei sich der Behälter (2) ferner von einem offenen oberen Ende (6) zu einem geschlossenen unteren Ende (7) erstreckt, wobei das offene obere Ende (6) einen vorbestimmten Durchmesser aufweist, der Behälter (2) ferner einen oberen Randabschnitt (8) am offenen oberen Ende (6) des Behälters (2) und einen Hauptabschnitt (9) umfasst, der einen abnehmenden Durchmesser des inneren Hohlraums (5) definiert, während er sich vom offenen oberen Ende (6) zum geschlossenen unteren Ende (7) des Behälters (2) nach unten erstreckt, wobei der Behälter (2) aus einem thermoplastischen Elastomer hergestellt ist
**dadurch gekennzeichnet, dass**
das thermoplastische Elastomer eine Menge an erneuerbarem thermoplastischem Elastomer umfasst, das als ein Elastomer definiert ist, das aus Biomasse aus einer lebenden Quelle stammt.

2. Menstruationstasse (1) nach einem der vorhergehenden Ansprüche, wobei das erneuerbare thermoplastische Elastomer aus Polyurethan-Elastomeren und/oder Styrol-Elastomeren ausgewählt ist.

3. Menstruationstasse nach Anspruch 1 oder 2, wobei das erneuerbare thermoplastische Elastomer zu etwa 20-100 Gew.-% des Gesamtgewichts des thermoplastischen Elastomers vorhanden ist.

4. Menstruationstasse (1) nach Anspruch 3, wobei das erneuerbare thermoplastische Elastomer zu etwa 30-90 Gew.-% des Gesamtgewichts des thermoplastischen Elastomers vorhanden ist.

5. Menstruationstasse (1) nach Anspruch 4, wobei das erneuerbare thermoplastische Elastomer zu etwa 40-80 Gew.-% des Gesamtgewichts des thermoplastischen Elastomers vorhanden ist.

6. Menstruationstasse (1) nach Anspruch 5, wobei das erneuerbare thermoplastische Elastomer zu etwa 50-70 Gew.-% des Gesamtgewichts des thermoplastischen Elastomers vorhanden ist.

7. Menstruationstasse nach einem der vorhergehenden Ansprüche, wobei das erneuerbare thermoplastische Elastomer nicht biologisch abbaubar ist.

8. Menstruationstasse nach einem der vorhergehenden Ansprüche, wobei der Hauptteil (9) des Behälters (2) eine Shore-Härte zwischen Shore A 20 und Shore A 70, bestimmt nach ISO 868 (Version 2003), aufweist.

9. Menstruationstasse (1) nach einem der vorhergehenden Ansprüche, wobei das thermoplastische Elastomer keinen Latex enthält.

10. Anordnung von Menstruationstassen nach einem der vorhergehenden Ansprüche, wobei die Anordnung mindestens zwei Behälter (2) mit unterschiedlichen Abmessungen umfasst, so dass ein erster der mindestens zwei Behälter (2) die Kapazität hat, eine größere Menge an Flüssigkeit aufzunehmen als ein zweiter der mindestens zwei Behälter (2).

11. Anordnung von Menstruationstassen nach einem der vorhergehenden Ansprüche, wobei die Anordnung mindestens zwei Behälter (2) mit unterschiedlichen Abmessungen umfasst, so dass ein erster der mindestens zwei Behälter (2) Abmessungen aufweist, die für Benutzerinnen mit einem niedrigen Gebärmutterhals und einem kürzeren Scheidenkanal geeignet ist, und ein zweiter der mindestens zwei Behälter (2) Abmessungen aufweist, die für Benutzerinnen mit einem hohen Gebärmutterhals und einem längeren Scheidenkanal geeignet ist.

12. Anordnung von Menstruationstassen nach einem der vorhergehenden Ansprüche, wobei die Anordnung mindestens zwei Behälter (2) mit unterschiedlichen Materialhärtegraden umfasst, so dass ein erster der mindestens zwei Behälter (2) einen Materialhärtegrad aufweist, der höher als ein Materialhärtegrad eines zweiten der mindestens zwei Behälter (2) ist.

13. Verfahren zum Herstellen einer Menstruationstasse (1) mit einem Design, das einen Behälter (2) mit einer Außenfläche (3) und einer Innenfläche (4) umfasst, wobei die Innenfläche (4) zumindest teilweise einen inneren Hohlraum (5) definiert, der zum Sammeln von Flüssigkeit geeignet ist, wobei sich der Behälter (2) ferner von einem offenen oberen Ende (6) zu einem geschlossenen unteren Ende (7) erstreckt, wobei das offene obere Ende (6) einen vorbestimmten Durchmesser aufweist, der Behälter (2) ferner einen oberen Randabschnitt (8) am offenen oberen Ende (6) des Behälters (2) und einen Hauptabschnitt (9) umfasst, der einen abnehmenden Durchmesser des inneren Hohlraums (5) definiert, während er sich vom offenen oberen Ende (6) zum geschlossenen unteren Ende (7) des Behälters (2) nach unten erstreckt, wobei das Verfahren Folgendes umfasst:
Formen der Menstruationstasse (1) mit einem Formwerkzeug, das für das Design der Menstruationstasse konfiguriert ist, unter Verwendung eines thermoplastischen Elastomers; und
Entnehmen der Menstruationstasse (1) aus dem Formwerkzeug;
**dadurch gekennzeichnet, dass**
das thermoplastische Elastomer, das zum Formen der Menstruationstasse (1) verwendet wird, eine Menge an erneuerbarem thermoplastischem Elastomer umfasst, das als ein thermoplastisches Elastomer definiert ist, das aus Biomasse aus einer lebenden Quelle stammt.

14. Verfahren nach Anspruch 13, wobei das erneuerbare thermoplastische Elastomer aus Polyurethan-Elastomeren und/oder Styrol-Elastomeren ausgewählt ist.

15. Verwendung eines thermoplastischen Elastomermaterials zur Herstellung einer Menstruationstasse, **dadurch gekennzeichnet, dass** das thermoplastische Elastomermaterial eine Menge an erneuerbarem thermoplastischem Elastomer umfasst, das als thermoplastisches Elastomer definiert ist, das aus Biomasse aus einer lebenden Quelle stammt, wobei das erneuerbare thermoplastische Elastomer aus Polyurethan-Elastomeren und/oder Styrol-Elastomeren ausgewählt ist.

## Revendications

1. Coupe menstruelle (1) comprenant un réceptacle (2) ayant une surface extérieure (3) et une surface intérieure (4), la surface intérieure (4) définissant au moins en partie une cavité intérieure (5) adaptée pour collecter un fluide, le réceptacle (2) s'étendant en outre d'une extrémité supérieure ouverte (6) à une extrémité inférieure fermée (7), l'extrémité supérieure ouverte (6) ayant un diamètre prédéterminé, le réceptacle (2) comprenant en outre une portion de bord supérieur (8) à l'extrémité supérieure ouverte (6) du réceptacle (2), et une portion principale (9) définissant un diamètre décroissant de la cavité intérieure (5) tout en s'étendant vers le bas depuis l'extrémité supérieure ouverte (6) jusqu'à l'extrémité inférieure fermée (7) du réceptacle (2), ledit réceptacle (2) étant fait d'un élastomère thermoplastique
**caractérisé en ce que**
ledit élastomère thermoplastique comprend un quantité d'élastomère thermoplastique renouvelable, défini comme un élastomère qui est d'origine de biomasse d'une source vivante.

2. Coupe menstruelle (1) selon l'une quelconque des revendications précédentes, où ledit élastomère thermoplastique renouvelable est sélectionné parmi des élastomères de polyuréthane et/ou des élastomères de styrène.

3. Coupe menstruelle selon la revendication 1 ou 2, où ledit élastomère thermoplastique renouvelable est présent à environ 20 à 100% en poids du poids total de l'élastomère thermoplastique.

4. Coupe menstruelle (1) selon la revendication 3, où ledit élastomère thermoplastique renouvelable est présent à environ 30 à 90% en poids du poids total de l'élastomère thermoplastique.

5. Coupe menstruelle (1) selon la revendication 4, où ledit élastomère thermoplastique renouvelable est présent à environ 40 à 80% en poids du poids total de l'élastomère thermoplastique.

6. Coupe menstruelle (1) selon la revendication 5, où ledit élastomère thermoplastique renouvelable est présent à environ 50 à 70% en poids du poids total de l'élastomère thermoplastique.

7. Coupe menstruelle selon l'une quelconque des revendications précédentes, où ledit élastomère thermoplastique renouvelable n'est pas biodégradable.

8. Coupe menstruelle selon l'une quelconque des revendications précédentes, où ladite portion principale (9) du réceptacle (2) a une dureté Shore entre Shore A 20 et Shore A 70 déterminée par ISO 868 (version 2003).

9. Coupe menstruelle (1) selon l'une quelconque des revendications précédentes, où l'élastomère thermoplastique ne contient pas de latex.

10. Ensemble de coupes menstruelles selon l'une quelconque des revendications précédentes, où ledit ensemble comprend au moins deux réceptacles (2) ayant des dimensions différentes de sorte qu'un premier des au moins deux réceptacles (2) a la capacité de recevoir une plus grande quantité de fluide qu'un deuxième des au moins deux réceptacles (2).

11. Ensemble de coupes menstruelles selon l'une quelconque des revendications précédentes, où ledit ensemble comprend au moins deux réceptacles (2) ayant des dimensions différentes de sorte qu'un premier des au moins deux réceptacles (2) a des dimensions qui sont adaptées aux utilisatrices qui ont un col de l'utérus bas et un canal vaginal plus court et qu'un deuxième des au moins deux réceptacles (2) a des dimensions qui sont adaptées aux utilisatrices qui ont un col de l'utérus haut et un canal vaginal plus long.

12. Ensemble de coupes menstruelles selon l'une quelconque des revendications précédentes, où ledit ensemble comprend au moins deux réceptacles (2) ayant des niveaux différents de dureté de matériau de sorte qu'un premier des au moins deux réceptacles (2) a un niveau de dureté de matériau qui est supérieur à un niveau de dureté de matériau d'un deuxième des au moins deux réceptacles (2).

13. Procédé de fabrication d'une coupe menstruelle (1) ayant une forme comprenant un réceptacle (2) ayant une surface extérieure (3) et une surface intérieure (4), la surface intérieure (4) définissant au moins en partie une cavité intérieure (5) adaptée pour collecter un fluide, le réceptacle (2) s'étendant en outre d'une extrémité supérieure ouverte (6) à une extrémité inférieure fermée (7), l'extrémité supérieure ouverte (6) ayant un diamètre prédéterminé, le réceptacle (2) comprenant en outre une portion de bord supérieur (8) à l'extrémité supérieure ouverte (6) du réceptacle (2), et une portion principale (9) définissant un diamètre décroissant de la cavité intérieure (5) tout en s'étendant vers le bas depuis l'extrémité supérieure ouverte (6) jusqu'à l'extrémité inférieure fermée (7) du réceptacle (2), le procédé comprenant :
mouler la coupe menstruelle (1) avec un outillage de moule configuré pour la forme de la coupe menstruelle, utilisant un élastomère thermoplastique; et extrayant la coupe menstruelle (1) du moulage ;
**caractérisé en ce que**
l'élastomère thermoplastique utilisé pour mouler la coupe menstruelle (1) comprend un quantité d'élastomère thermoplastique renouvelable, défini comme un élastomère thermoplastique qui est d'origine de biomasse d'une source vivante.

14. Procédé selon la revendication 13, où ledit élastomère thermoplastique renouvelable est sélectionné parmi des élastomères de polyuréthane et/ou des élastomères de styrène.

15. Utilisation d'un matériau élastomère thermoplastique pour fabriquer une coupe menstruelle, **caractérisée en ce que** ledit matériau élastomère thermoplastique comprend un quantité d'élastomère thermoplastique renouvelable, défini comme un élastomère thermoplastique qui est d'origine de biomasse d'une source vivante, ledit élastomère thermoplastique renouvelable étant sélectionné parmi des élastomères de polyuréthane et/ou des élastomères de styrène.
